# EUROPEAN PATENT APPLICATION

(11) **EP 1 270 718 A1**
(43) Date of publication of application: **02.01.2003**
(21) Application number: 01917611.4
(22) Date of filing: 29.03.2001
(51) Int. Cl.: C12N 5/00, C12M 3/00, G06T 1/00

(54) **CELL CULTURING METHOD AND DEVICE**

(30) Priority: 31.03.2000 JP 2000099684
(71) Applicant: Japan Tissue Engineering Co., Ltd., Gamagori-shi, Aichi 443-0022 (JP); Taya, Masahito, Toyonaka-shi, Osaka 561-0893 (JP)
(72) Inventor: TAYA, Masahito, Toyonaka-shi, Osaka 561-0893 (JP); KINOOKA, Masahiro, Toyonaka-shi, Osaka 560-0005 (JP); UMEGAKI, Ryota, Toyonaka-shi, Osaka 560-0011 (JP)
(74) Representative: Reinhard - Skuhra - Weise & Partner
(86) International application number: JP0102646
(87) International publication number: WO01075070

(57) **Abstract**

A cell culture device which facilitates a culture operation while maintaining conditions suited for culture of cells. A cell culture device 11 comprises first and second culture units 12a and 12b, a cell feed unit 14, a liquid feed unit 15, a liquid waste tank 16, a gas exchange unit 18, and a control unit 101. Cells are cultured in the first and second culture units 12a and 12b. A CCD camera 22 provides image data of the cells to the control unit 101. The control unit calculates concentration of the cells from the image data and determines timings to replace a culture medium and perform subculture in accordance with the concentration.

## Description

### Technical Field

The present invention relates to a method for culturing cells, and more specifically, it relates to a method for culturing anchorage-dependent cells by use of a culture chamber.

### Background Art

Heretofore, when anchorage-dependent cells are cultured in vitro, almost all culturing operations have been performed manually. The main culturing operations include an operation in which an old culture medium (waste culture medium) in a culture chamber is replaced with a new culture medium and a subculture operation. In the subculture operation, when cells reach a confluent state in a culture chamber, the cells are distributed over a number of other culture media and grown. The term "confluent state" refers to a state that cells cover a substantial area of the bottom of a culture chamber in a single layer.

The culture medium replacing operation must be performed before the most consumable component among a variety of components in a culture medium is consumed completely. This is important for the sake of preventing growth of cells from being influenced by lack of the consumable component. The subculture operation is performed every time cells become confluent state in a culture chamber. Timings to perform the culture medium replacing operation and the subculture operation have been determined based on experiences of an operator.

The culture medium replacing operation is carried out in a clean bench. More specifically, a new culture medium is heated to 37°C in advance. A waste culture medium is taken out of a culture chamber with which cells are in adhering by use of a pipette. Then, the preheated, new culture medium is injected into the culture chamber by use of a pipette.

The subculture operation is also carried out in a clean bench. More specifically, firstly, a waste culture medium is removed from a culture chamber. As required, calcium ions are rinsed from the culture chamber. Then, a predetermined concentration of trypsin is added to the culture chamber so as to cause cells to detach from the bottom of the culture chamber. Then, a trypsin inhibitor is added so as to stop the cells from detaching from the bottom of the chamber. A cell suspension containing the trypsin, trypsin inhibitor and cells which have detached from the bottom of the chamber was transferred from the culture chamber to a centrifugal tube by use of a pipette. A centrifugal separator was operated at a rotational speed of around 1,000 r.p.m. so as to precipitate only the cells. A supernatant containing the trypsin and the trypsin inhibitor is removed from the centrifugal tube. A new culture medium is injected into the centrifugal tube so as to suspend the cells again. Then, the cell suspension is distributed over a plurality of culture chambers.

In a'conventional method for culturing cells, almost all culturing operations require some manual works (for example, to lean a culture chamber upon extraction by use of a pipette) and are complicated accordingly. Further, timings to perform the culture medium replacing operation and the subculture operation based on experiences are very rough.

### Disclosure of the Invention

It is an object of the present invention to provide a method and device for culturing cells which is capable of carrying out culturing operations easily while maintaining conditions suitable for culturing cells.

To achieve the above object, a first aspect of the present invention provides a method for monolayer-culturing anchorage-dependent cells in a culture container. The culturing method includes the steps of taking a picture of anchorage-dependent cells in the culture container so as to produce image data, calculating at least one status value of the number of cells adhered in the culture container, the concentration of the adhered cells, an area occupied by the adhered cells and an occupancy rate of the adhered cells based on the image data, determining timing to perform at least one operation selected from a culture medium replacing operation and a subculture operation based on the at least one status value, and performing the selected operation(s) at the determined timing.

A second aspect of the present invention provides a cell culture device for monolayer-culturing anchorage-dependent cells in a culture container while performing at least one operation selected from a culture medium replacing operation and a subculture operation. The cell culture device includes a culture container, a photographic unit which takes a picture of anchorage-dependent cells in the culture container so as to produce image data of the cells, and a control unit which is connected to the photographic unit so as to process the image data. The control unit calculates at least one of the number of cells adhered in the culture container, the concentration of the adhered cells in the culture container, an area in the culture container which is occupied by the adhered cells and a proportion of the adhered cells in the culture container and determines timing to perform the operations based on the result of the calculation.

A third aspect of the present invention provides a system for monolayer-culturing anchorage-dependent cells. The system includes a first culture dish which partitions a first culture medium chamber in which cells are cultured, a second culture dish which partitions a second culture medium chamber connected to the first culture medium chamber and having a larger base area than a base area of the first culture medium chamber, a photographic unit which takes a picture of cells in the first and second culture medium chambers so as to produce image data thereof, a control unit which is connected to the photographic unit so as to process the image data, a transfer mechanism which is connected to the control unit so as to transfer cells in the first culture chamber to the second culture chamber in accordance with a command from the control unit, a feed pump which is connected to the control unit so as to feed a culture medium and the cells to the culture chambers selectively in accordance with a command from the control unit, and a discharge pump which is connected to the control unit so as to discharge a waste culture medium from the culture chambers in accordance with a command from the control unit. The control unit calculates the at least one of the number of cells adhered in the culture container, the concentration of the adhered cells in the culture container, an area in the culture container which is occupied by the adhered cells and a proportion of the adhered cells in the culture container and controls the transfer mechanism, feed pump and the discharge pump based on the result of the calculation.

A fourth aspect of the present invention provides a recording medium containing a computer readable program for monolayer-culturing anchorage-dependent cells in a culture container while performing at least one operation selected from a culture medium replacing operation and a subculture operation. The program executes a method includes the steps of inputting image data of anchorage-dependent cells in the culture container by means of a photographic unit, calculating the number of adhered cells, the concentration of the adhered cells, an area occupied by the adhered cells or a proportion of the adhered cells based on the image data, determining timing to perform at least one operation selected from a culture medium replacing operation and a subculture operation based on the result of the calculation, and performing the operation.

A fifth aspect of the present invention provides a culture container for monolayer-culturing anchorage-dependent cells while performing a subculture operation. The culture container is a polygonal, tubular container having a plurality of sides and a shaft extending in a nearly horizontal direction. The culture container accommodates a plurality of culture dishes located on inner surfaces of its plurality of sides. The plurality of culture dishes is a first culture dish having a first area and a second culture dish having a second area which is larger than the first area. The culture container has a position changing device for selectively moving one of the plurality of culture dishes to a low position.

### Brief Description of the Drawings

Fig. 1 is a schematic diagram of a cell culture device according to a first embodiment of the present invention.
Fig. 2 is a perspective view of a first culture unit in Fig. 1.
Fig. 3 is a sectional side view of the first culture unit in Fig. 2.
Fig. 4A is a perspective view of a first culture dish in Fig. 3.
Fig. 4B is a perspective view of a clip in Fig. 3.
Fig. 5 is a perspective view of a second culture dish in Fig. 1.
Fig. 6 is a schematic block diagram of a control unit of the cell culture device in Fig. 1.
Figs. 7 and 8 are flowcharts of steps for controlling the present invention.
Fig. 9 is a schematic diagram of a cell culture device according to a second embodiment of the present invention.
Fig. 10A is a perspective view of a first culture unit in Fig. 9.
Fig. 10B is a sectional view taken along the line 10b-10b of the culture container in Fig. 10A.
Fig. 11 is a schematic block diagram of a control unit of the cell culture device in Fig. 9.
Fig. 12A is a perspective view of a jig for extracting tissue.
Fig. 12B is a sectional view of the jig in Fig. 12A.

### Best Mode for Carrying out the Invention

### (First Embodiment)

Hereinafter, a cell culture device 11 according to a first embodiment of the present invention will be described.

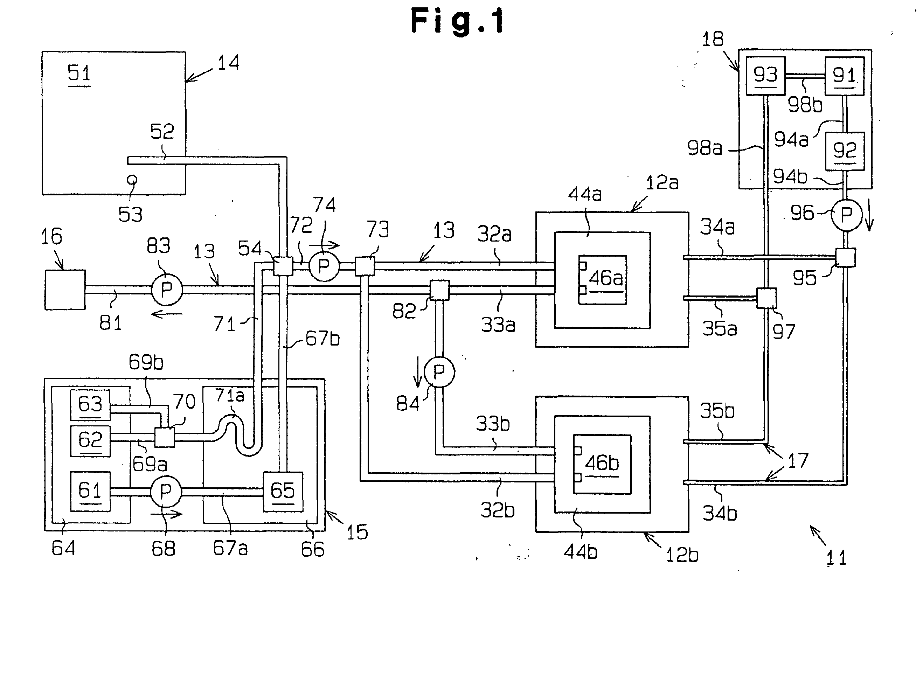

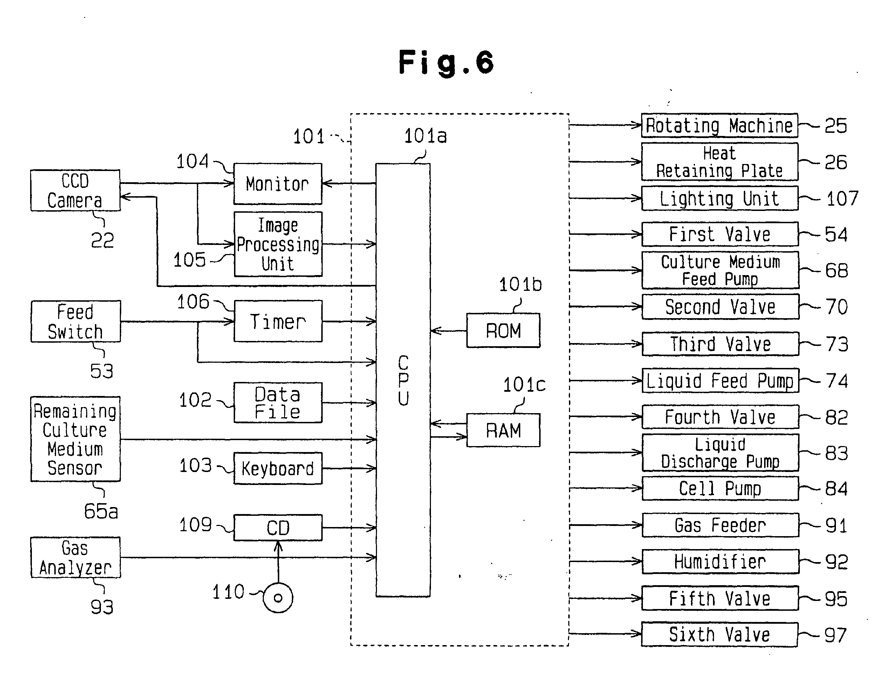

As shown in Fig. 1, the cell culture device 11 comprises a first culture unit 12a, a second culture unit 12b, a cell feed unit 14, a liquid feed unit 15, a liquid waste tank 16, a gas exchange unit 18 and a control unit (shown in Fig. 6). Liquid pipes 13 connect the culture units 12a and 12b to the cell feed unit 14, the liquid feed unit 15 and the liquid waste tank 16. Gas pipes 17 connect the culture units 12a and 12b to the gas exchange unit 18.

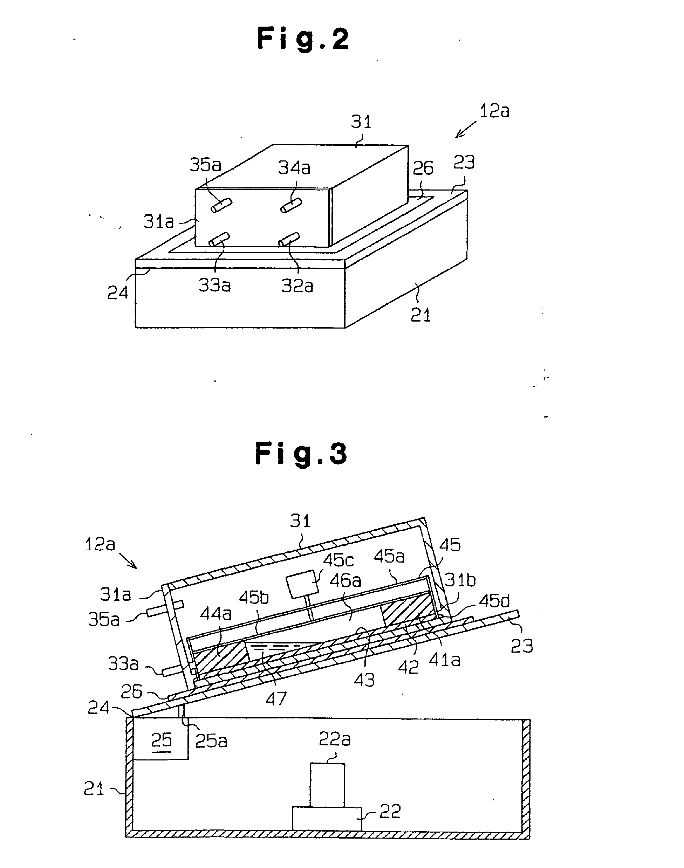

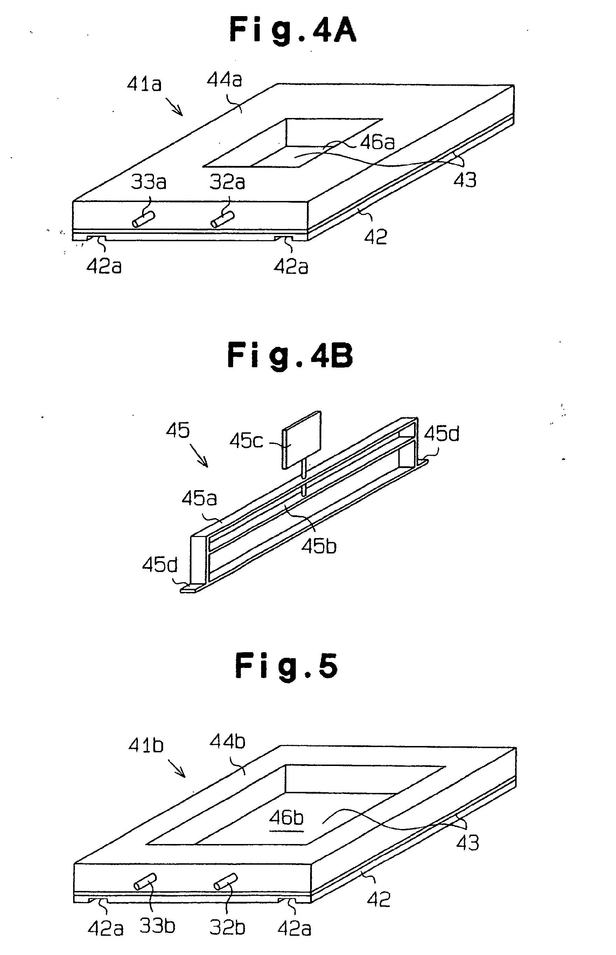

As shown in Figs. 2 and 3, the first culture unit 12a has a stage 21. At the center of the bottom of the stage 21, a charge coupled device (CCD) camera 22 is disposed. A lens 22a of the CCD camera 22 faces upward.

The stage 21 has an opening at the top. Preferably, the top opening is selectively closed by a stage plate 23 which is made of a transparent material such as glass or acrylic. The stage plate 23 is rotatably connected to an upper edge of the stage 21 by means of a hinge 24. In the stage 21, a rotating machine 25 having a rotating bar 25a which can be displaced in an axial direction and makes contact with the stage plate 23 is disposed. The rotating machine 25 changes an inclination angle of the stage plate 23 by displacing the rotating bar 25a. On the upper surface of the stage plate 23, a heat retaining plate 26 which is made of a transparent material is preferably fixed. A culture container 31 is mounted on the heat retaining plate 26 and retained at a predetermined temperature (for example, at 37°C).

The culture container 31 is preferably made of a transparent synthetic resin. Above the culture container 31, a lighting unit (not shown) for lighting the culture container 31 is disposed. The culture container 31 has an opening on a side close to the hinge 24, and the opening is sealed by a cover 31a. At lower ends of internal walls of front and back sides of the culture container 31, projections 31b which extend inwardly are formed.

To the cover 31a of the first culture unit 12a, a first liquid feed pipe 32a, a first liquid discharge pipe 33a, a first gas feed pipe 34a and a first gas discharge pipe 35a are connected. The first gas feed pipe 34a and the first gas discharge pipe 35a are disposed above the first liquid feed pipe 32a and the first liquid discharge pipe 33a, respectively.

As shown in Fig. 3, the first culture unit 12a has a first culture dish 41a which is placed at the bottom of the culture container 31. The first culture dish 41a comprises a base plate 42, a hydrophilic film 43, a first barrier 44a and a clip 45.

The base plate 42 is preferably made of a transparent synthetic resin such as polycarbonate. On the underside of the base plate 42, a pair of grooves 42a which extend parallel to each other (refer to Fig. 4A) are formed. The transparent, hydrophilic resin film 43 is disposed on the upper surface of the base plate 42. The first barrier 44a having an opening in a central portion is placed on the hydrophilic resin film 43. The first barrier 44a is preferably made of a hydrophobic resin such as a silicone resin. The first barrier 44a and the hydrophilic film 43 partition a first culture chamber 46a. In the first culture chamber 46a, anchorage-dependent cells are cultured. The first liquid feed pipe 32a and the first liquid discharge pipe 33a penetrate a wall of the first barrier 44a and communicate with the first culture chamber 46a. The base plate 42, the hydrophilic resin film 43 and the first barrier 44a are clipped by a pair of clips 45 (only one of them is shown) shown in Fig. 4B.

The clip 45 comprises a long, slim frame 45a, a press plate 45b which slides inside the frame 45a, and a press screw 45c which is connected to the press plate 45b. The base plate 42 is inserted between the press plate 45b and the lower plate of the frame 45a so as to fit the lower plate of the frame 45a into the groove 42a of the base plate 42, and the press screw 45c is then fastened securely, thereby fixing the clip 45 to the first barrier 44a. The frame 45a has a pair of engagement lugs 45d which extend outwardly at both ends in its longitudinal direction. The engagement lugs 45d each engage the corresponding projections 31b of the culture container 31.

The structure of the second culture unit 12b is almost the same as that of the first culture unit 12a except for the following points.

To a cover 31a of the second culture unit 12b, a second liquid feed pipe 32b, a second liquid discharge pipe 33b, a second gas feed pipe 34b and a second gas discharge pipe 35b are connected (refer to Figs. 1 and 5). The second gas feed pipe 34b and the second gas discharge pipe 35b are disposed above the second liquid feed pipe 32b and the second liquid discharge pipe 33b, respectively. The second culture unit 12b has a second culture dish 41b which is shown in Fig. 5. The second culture dish 41b includes a second barrier 44b. The second barrier 44b partitions a second culture chamber 46b which has a larger base area than that of the first culture chamber 46a. In other words, four walls of the second barrier 44b are smaller in width than those of the first barrier 44a. The second liquid feed pipe 32b and the second liquid discharge pipe 33b penetrate a wall of the second barrier 44b and communicate with the second culture chamber 46b.

As shown in Fig. 1, the cell feed unit 14 comprises a clean bench 51 which is schematically shown, a cell feed pipe 52 which has an opened tip inside the clean bench 51, and a feed switch 53 which is disposed in the vicinity of the tip of the cell feed pipe 52. The other end of the cell feed pipe 52 is connected to a first electrically driven valve 54.

The liquid feed unit 15 for feeding a culture medium and a cell release agent includes a simple refrigerator 64 and an incubator 66. The inside of the simple refrigerator 64 is kept at about 5°C. The inside of the incubator 66 is kept at about 37°C. The simple refrigerator 64 houses a cold culture medium tank 61, a release agent tank 62 and a release agent inhibitor tank 63. The incubator 66 houses a hot culture medium tank 65. The cold culture medium tank 61 stores a culture medium 47, the release agent tank 62 stores a cell release agent such as trypsin, and the release agent inhibitor tank 63 stores a release agent inhibitor such as a trypsin inhibitor. The hot culture medium tank 65 stores a culture medium 47 which is kept at 37°C. The hot culture medium tank 65 is provided with a remaining culture medium sensor which is not shown.

The cold culture medium tank 61 and the hot culture medium tank 65 are connected to each other via a first culture medium feed pipe 67a. The first culture medium feed pipe 67a has a culture medium feed pump 68. To the hot culture medium tank 65, a second culture medium feed pipe 67b is connected. The second culture medium feed pipe 67b is connected to the first electrically driven valve 54. The release agent tank 62 is connected to a second electrically driven valve 70 via a release agent pipe 69a. The release agent inhibitor tank 63 is connected to the second electrically driven valve 70 via a release agent inhibitor pipe 69b. A subculture pipe 71 is connected to the second electrically driven valve 70 and the first electrically driven valve 54. The subculture pipe 71 has a heating portion 71a having a relatively large surface area. The heating portion 71a is placed within the incubator 66. Thereby, a liquid which passes through the heating portion 71a is heated smoothly.

The first electrically driven valve 54 is connected to a liquid feed pump 74 and a third electrically driven valve 73 via a liquid feed pipe 72. The third electrically driven valve 73 is connected to the first and second liquid feed pipes 32a and 32b.

The liquid waste tank 16 which temporarily stores a liquid discharged from the culture container 31 is connected to a liquid discharge pipe 81. The liquid discharge pipe 81 is connected to a fourth electrically driven valve 82 and has a liquid discharge pump 83 somewhere in the middle. The fourth electrically driven valve 82 is connected to the first and second liquid discharge pipes 33a and 33b. The liquid waste tank 16, the liquid discharge pipe 81, the fourth electrically driven valve 82, the liquid discharge pump 83 and the first and second liquid discharge pipes 33a and 33b constitute a liquid discharge assembly. In the middle of the second liquid discharge pipe 33b, a cell transfer pump 84 for transferring a cell suspension in the first culture unit 12a to the second culture unit 12b is provided.

The gas exchange unit 18 includes a gas feeder 91, a humidifier 92 and a gas analyzer 93. The gas feeder 91 feeds germ-free carbon dioxide gas and oxygen gas from a carbon dioxide gas bomb and an oxygen bomb which are not shown to the humidifier 92. The gas feeder 91 has a ventilating function. In other words, the gas feeder 91 takes in germ-free air through a filter which is not shown and feeds the germ-free air to the humidifier 92 or exhausts the germ-free air from the humidifier 92. The humidifier 92 generates steam so as to moisten the received gas.

A first gas supply pipe 94a connects the gas feeder 91 and the humidifier 92 to each other. To the humidifier 92, a second gas supply pipe 94b is connected. The second gas supply pipe 94b is connected to a fifth electrically driven valve 95 and has a gas circulating pump 96 somewhere in the middle. The fifth electrically driven valve 95 is connected to the first and second gas feed pipes 34a and 34b. The first and second gas discharge pipes 35a and 35b are connected to a sixth electrically driven valve 97. The sixth electrically driven valve 97 is connected to a first gas circulating pipe 98a.

The first gas circulating pipe 98a is connected to the gas analyzer 93. The gas analyzer 93 analyzes gas discharged from the culture container 31 of the first culture unit 12a or the second culture unit 12b and provides data on constituents of the gas (data on concentration of carbon dioxide gas, concentration of oxygen and humidity) to the control unit.

The gas analyzer 93 is connected to a second gas circulating pipe 98b. The second gas circulating pipe 98b is connected to the gas feeder 91. A portion of gas analyzed in the gas analyzer 93 is returned to the gas feeder 91 so as to be recycled.

As shown in Fig. 6, a control unit 101 comprises a CPU 101a, a ROM 101b and a RAM 101c. A data file 102, a keyboard 103, a monitor 104, an image processing unit 105, a timer 106 and a CD-ROM drive 109 are connected to the control unit 101. A CD-ROM 110 contains control program data for operating the cell culture device 11. 'The CD-ROM drive 109 reads the program data recorded on the CD-ROM 110 and provides it to the CPU 101a. The CPU 101a loads the ROM 101b such as an EEPROM with the program data. Alternatively, the ROM 101b may contain the control program of the cell culture device 11 in advance. The CPU 101a performs a variety of computations as of an image matching process in accordance with the control program stored in the ROM 101b. The RAM 101c temporarily stores results of computations performed by the CPU 101a.

The data file 102 is constituted by a storage device such as a hard disk drive and stores predetermined image data such as cell pattern data of anchorage-dependent cells and threshold value data in a remaining culture medium sensor 65a and the gas analyzer 93. The CPU 101a compares the cell pattern data with image data photographed by the CCD camera 22 and calculates concentration of adhered cells in the image data. Further, the CPU 101a also determines whether remaining culture medium data provided from the remaining culture medium sensor 65a and data about concentration of carbon dioxide gas, concentration of oxygen and humidity which are provided from the gas analyzer 93 match the threshold value data.

A command for starting, terminating or correcting the control program is provided to the CPU 101a via the keyboard 103. The monitor 104 displays operation data of the cell culture device 11 and image data photographed by the CCD camera 22. The monitor 104 is capable of switching between image data to be displayed of the first culture unit 12a and the second culture unit 12b.

The image processing unit 105 performs normalization and feature extraction. The unit 105 cuts extracted partial section data from image data provided from the CCD camera 22 and provides it to the CPU 101a. In other words, the CPU 101a takes in the partial section data of the image data via the image processing unit 105.

When the feed switch 53 is switched ON, the CPU 101a switches the first and third electrically driven valves 54 and 73 so as to communicate the cell feed pipe 52, the liquid feed pipe 72 and the first liquid feed pipe 32a with each other. The CPU 101a drives the liquid feed pump 74 while the feed switch 53 being pressed down. Thereby, a cell suspension is fed to the first culture chamber 46a via the cell feed pipe 52. When time during which the feed switch 53 has been continuously pressed down exceeds a predetermined limit time, the CPU 101a stops the liquid feed pump 74. In response to press of the feed switch 53, the timer 106 starts measurement of culture time and provides the culture time data to the CPU 101a.

The remaining culture medium sensor 65a measures a remaining culture medium 47 in the hot culture medium tank 65 and provides the remaining culture medium data to the CPU 101a. The CPU 101a determines whether the remaining culture medium data falls within a predetermined hot culture medium amount range. When the remaining culture medium data is smaller than the hot culture medium amount range, the CPU 101a drives the culture medium feed pump 68 so as to transfer a culture medium 47 in the cold culture medium tank 61 to the hot culture medium tank 65. Meanwhile, when the remaining culture medium data is larger than the hot culture medium amount range, the CPU 101a stops the culture medium feed pump 68.

The gas analyzer 93 analyzes gas received via the first gas circulating pipe 98a and generates data on concentration of carbon dioxide gas, data on concentration of oxygen, and data on humidity. The CPU 101a determines whether the carbon dioxide gas concentration data falls within a predetermined carbon dioxide gas concentration range (for example, about 5 to 10%), determines whether the oxygen concentration data falls within a predetermined oxygen gas concentration range (for example, about 15 to 25%), and determines whether the humidity data falls within a predetermined humidity range.

When the carbon dioxide gas concentration data is lower than the carbon dioxide gas concentration range, the CPU 101a activates the gas feeder 91 so as to feed a carbon dioxide gas, while when the carbon dioxide gas concentration data is higher than the carbon dioxide gas concentration range, the CPU 101a deactivates the gas feeder 91 so as to stop feeding the carbon dioxide gas. When the oxygen concentration data is lower than the oxygen gas concentration range, the CPU 101a activates the gas feeder 91 so as to feed an oxygen gas, while when the oxygen concentration data is higher than the oxygen gas concentration range, the CPU 101a deactivates the gas feeder 91 so as to stop feeding the oxygen gas. When the humidity data is lower than the humidity range, the CPU 101a activates the humidifier 92 so as to generate steam, while when the humidity data is higher than the humidity range, the CPU 101a deactivates the humidifier 92 so as to stop feeding the steam.

To increase an inclination angle of the stage plate 23, the CPU 101a continues to send an extension signal to the rotating machine 25 over a predetermined time period so as to extend the rotating bar 25a. On the other hand, to decrease the inclination angle of the stage plate 23 (or put the stage plate 23 back to a horizontal position), the CPU 101a drives the rotating machine 25 so as to retract the rotating bar 25a. The CPU 101a switches ON/OFF positions of the heat retaining plate 26 and a lighting unit 107 in the first culture unit 12a or the second culture unit 12b in synchronization with switching of the CCD camera 22.

The CPU 101a sends a switch signal to the second electrically driven valve 70 so as to communicate the release agent pipe 69a or the release agent inhibitor pipe 69b with the subculture pipe 71. The CPU 101a sends a switch signal to the fourth electrically driven valve 82 so as to communicate the liquid discharge pipe 81 with the first liquid discharge pipe 33a, communicate the liquid discharge pipe 81 with the second liquid discharge pipe 33b or communicate the first liquid discharge pipe 33a with the second liquid discharge pipe 33b. The CPU 101a sends a drive signal to the liquid discharge pump 83 so as to discharge a liquid in the culture container 31 to the liquid waste tank 16 through the liquid discharge pipe 81. The CPU 101a sends a drive signal to the cell transfer pump 84 so as to transfer a cell suspension in the first culture chamber 46a to the second culture chamber 46b.

The CPU 101a switches the fifth and sixth electrically driven valves 95 and 97 simultaneously in synchronization with switching of the CCD camera 22. Thereby, the second gas supply pipe 94b, the first gas feed pipe 34a, the first gas discharge pipe 35a and the first gas circulating pipe 98a are communicated with each other simultaneously, and the second gas supply pipe 94b, the second gas feed pipe 34b, the second gas discharge pipe 35b and the first gas circulating pipe 98a are communicated with each other simultaneously.

Operations of the cell culture device 11 will be described.

When cells are cultured, firstly, an operator assembles germ-free culture containers 31. More specifically, as shown in Figs. 2 to 4, liquid feed pipes 32a and 32b, liquid discharge pipes 33a and 33b, gas feed pipes 34a and 34b and gas discharge pipes 35a and 35b are caused to penetrate at predetermined positions of covers 31a, and the liquid feed pipes 32a and 32b and the liquid discharge pipes 33a and 33b are caused to penetrate at predetermined positions of first and second barriers 44a and 44b.

Then, first and second culture dishes 41a and 41b are assembled. More specifically, a hydrophilic film 43 is laminated on a base plate 42, and the first barrier 44a or the second barrier 44b is laminated on the hydrophilic film 43. Thereafter, the operator fixes the first barrier 44a and the second barrier 44b to corresponding base plates 42 by use of clips 45 so as to prevent culture media 47 in first and second culture chambers 46a and 46b from leaking.

The first and second culture dishes 41a and 41b are placed on the bottoms of the culture containers 31. Engagement lugs 45d engage corresponding projections 31b, and the culture container 31 is sealed by the cover 31a. The culture container 31 is placed in the center of a heat retaining plate 26. A given amount of culture medium 47 is filled in a cold culture medium tank 61, a given amount of cell release agent is filled in a release agent tank 62, a given amount of release agent inhibitor is filled in a release agent inhibitor tank 63, given amounts of carbon dioxide gas and oxygen gas are filled in a carbon dioxide gas bomb and oxygen gas bomb of the gas feeder 91, and a given amount of water is filled in a humidifier 92. Then, the operator gives a command to start culture to a CPU 101a by means of a keyboard 103.

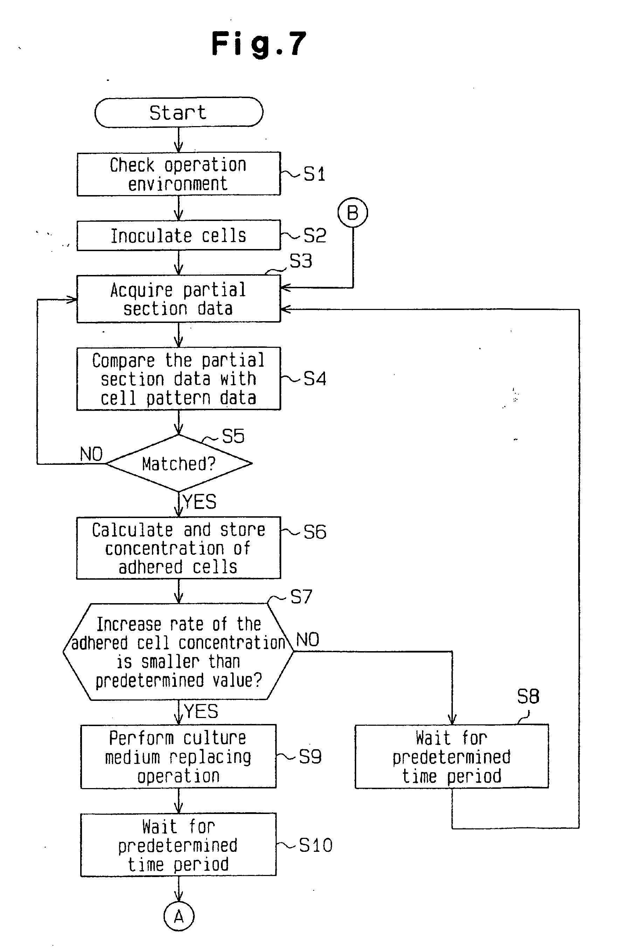

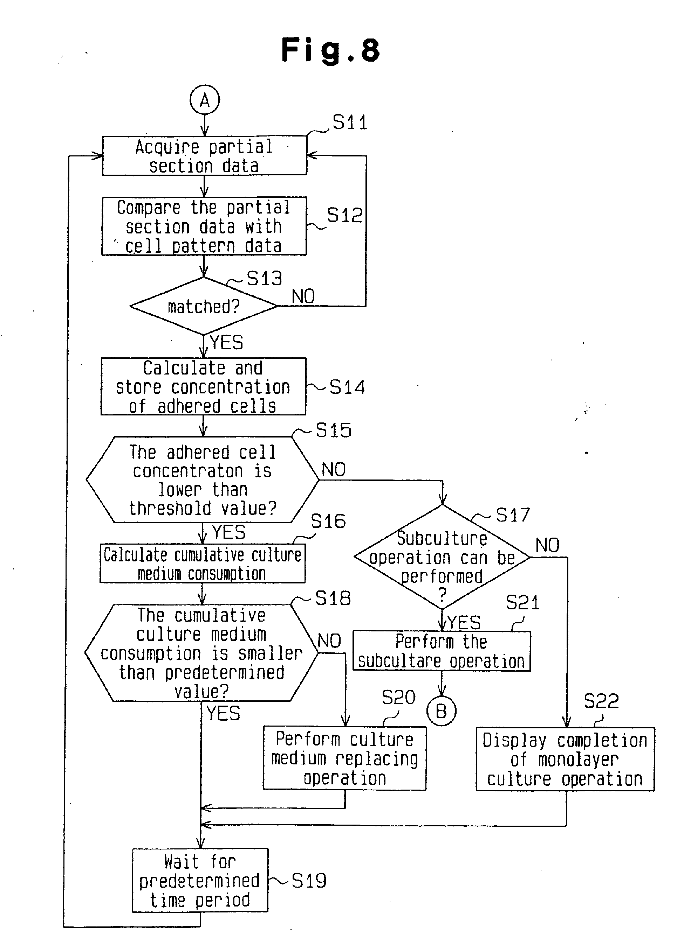

Processes carried out by the CPU 101a will be described with reference to flowcharts of Figs. 7 and 8.

In response to the start command, the CPU 101a checks an operation environment of the cell culture device 11 in step S1 shown in Fig. 7. More specifically, the CPU 101a activates not only a simple refrigerator 64, an incubator 66, a gas analyzer 93, a gas circulating pump 96 and a remaining culture medium sensor 65a but also a CCD camera 22 in a first culture unit 12a, a heat retaining plate 26 and a lighting unit 107.

Then, the lighting unit 107 lights the first culture dish 41a, and a picture of the first culture chamber 46a is taken by the CCD camera 22 and then displayed on a monitor 104. Further, the CPU 101a also displays operation environment data of units constituting the cell culture device 11. At this point, the CPU 101a drives a culture medium feed pump 68 based on remaining culture medium data provided from the remaining culture medium sensor 65a so as to transfer a given amount of culture medium 47 to a hot culture medium tank 65.

Meanwhile, the CPU 101a sends a switch signal to fifth and sixth electrically driven valves 95 and 97 so as to communicate a second gas supply pipe 94b, a first gas feed pipe 34a, a first gas discharge pipe 35a and a first gas circulating pipe 98a with each other and feeds gas to the culture container 31 of the first culture unit 12a. The CPU 101a activates the gas feeder 91 and the humidifier 92 in accordance with analysis data of the gas analyzer 93 so as to adjust constituents of the gas. After preparation of the cell culture device 11 is completed, the CPU 101a provides operation environment data or a preparation-completed signal to a monitor 104 so as to display data notifying the completion of the preparation on the monitor 104.

The CPU 101a performs inoculation of cells in step S2. More specifically, the operator prepares a cell suspension of anchorage-dependent cells in a clean bench 51 manually in advance. The operator immerses the tip of a cell feed pipe 52 in the cell suspension and then presses down a feed switch 53. In response to this, the CPU 101a sends a switch signal to first, third and fourth electrically driven valves 54, 73 and 82. Thereby, the cell feed pipe 52, the liquid feed pipe 72 and the first liquid feed pipe 32a are communicated with each other, and the fourth electrically driven valve 82 is closed. Then, the CPU 101a drives a liquid feed pump 74 while the feed switch 53 being pressed down, thereby feeding the cell suspension to the first culture chamber 46a of the first culture unit 12a.

A control unit 101 acquires partial section data in step S3. More specifically, cells (anchorage-dependent cells) adhered to the bottom of a first culture dish 41a are photographed by the CCD camera 22, and the control unit 101 provides image data of the photographed cells to an image processing unit 105. The image processing unit 105 performs character extraction of the image data so as to generate partial section data and provides the acquired data to the CPU 101a. In step S4, the CPU 101a compares the partial section data with cell pattern data stored in a date file 102. The CPU 101a determines in step S5 whether the partial section data matches the cell pattern data. When they do not match each other, the CPU 101a returns to step S3, while when they match each other, the CPU 101a proceeds to step S6. In step S6, the CPU 101a calculates density of the anchorage-dependent cells (adhered cell concentration) based on the partial section data and stores the calculated value in a RAM 101c.

In step S7, the CPU 101a calculates an increase rate from a difference between the adhered cell concentration and the adhered cell concentration calculated last time. When the concentration calculated last time is not stored in the RAM 101c, the calculation of the increase rate is made with the last concentration being zero. Then, the CPU 101a compares the increase rate with a preset value (predetermined value) stored in a ROM 101b. When the increase rate is larger than or equal to the preset value, the CPU 101a waits for a predetermined amount of time (step S8) and then returns to step S3. When the increase rate is smaller than the preset value, the CPU 101a performs a culture medium replacing operation (replacement of culture medium after adhesion) in step S9. The preset value is preferably 0 or nearly 0.

In step S9, the CPU 101a drives the fourth electrically driven valve 82 so as to communicate the first liquid discharge pipe 33a with a liquid discharge pipe 81. The CPU 101a drives a liquid discharge pump 83 so as to discharge a waste culture medium 47 in the first culture chamber 46a to a liquid waste tank 16 and also slowly extends a rotating bar 25a. Thereby, the culture container 31 is inclined (as shown in Fig. 3), so that the waste culture medium 47 and cells unable to adhere to a hydrophilic film 43 are pumped out by means of the liquid discharge pump 83 via the first liquid discharge pipe 33a. Thereby, only cells adhered to the top surface of the hydrophilic film 43 remain in the first culture chamber 46a.

When stopping the liquid discharge pump 83, the CPU 101a closes the fourth electrically driven valve 82. Subsequently, the CPU 101a retracts the rotating bar 25a and puts a stage plate 23 back to a horizontal position. The CPU 101a switches the first and third electrically driven valves 54 and 73 so as to communicate a second culture medium feed pipe 67b, the liquid feed pipe 72 and the first liquid feed pipe 32a with each other. The CPU 101a drives the liquid feed pump 74 so as to feed a hot culture medium 47 from a hot culture medium tank 65 to the first culture chamber 46a. Then, the CPU 101a stops the liquid feed pump 74 and closes the first and third electrically driven valves 54 and 73.

After waiting for a predetermined amount of time in step S10, the CPU 101a carries out steps S11 to S14. Steps S11 to S14 are similar processes to steps S3 to S6. In step S15, the CPU 101a compares adhered cell concentration calculated in step S14 this time with a threshold value stored in the data file 102. When the adhered cell concentration is lower than the threshold value, the CPU 101a proceeds to step S16. When the adhered cell concentration is higher than the threshold value, the CPU 101a proceeds to step S17. As the threshold value, adhered cell concentration in a nearly confluent state is set, for example.

In step S16, the CPU 101a calculates the amount of culture medium consumed by anchorage-dependent cells within a predetermined time period and a cumulative culture medium consumption which indicates a current state of the culture medium 47 from the adhered cell concentration calculated this time with the last cell concentration. More specifically, the CPU 101a, firstly, reads a culture medium consumption rate per unit time of anchorage-dependent cells from the data file 102. The culture medium consumption rate is preferably predetermined for the most consumable component among components in the culture medium 47 by experiments and stored in the data file 102. The CPU 101a calculates an average of the adhered cell concentration of this time and the last cell concentration and also calculates an amount of time elapsed between the last calculation time (culture time) and the current time. The CPU 101a integrates the culture medium consumption rate, the average concentration and the elapsed time so as to determine an amount of culture medium consumed within the elapsed time. The amount of consumed culture medium is stored in the RAM 101c. In addition, the CPU 101a calculates a cumulative culture medium consumption value between the last culture medium replacement and the current time.

In step S18, the CPU 101a compares the cumulative culture medium consumption with a predetermined amount stored in the data file 102. When the cumulative culture medium consumption is smaller than the predetermined amount, the CPU 101a waits for a predetermined amount of time in step S19 and then returns to step S11. When the cumulative culture medium consumption is greater than or equal to the predetermined amount, the CPU 101a performs a culture medium replacing operation in step S20. The predetermined amount is an amount which is nearly equivalent to or smaller than the initial amount of the culture medium 47 fed to the first culture chamber 46a, for example. Further, the predetermined amount may be an amount which is nearly equivalent to or smaller than an amount of given components present in the initial amount of the culture medium 47.

When the adhered cell concentration is higher than or equal to the threshold value in step S15, the CPU 101a determines in step S17 whether a subculture operation can be performed in either the first culture unit 12a or the second culture unit 12b. For example, the CPU 101a determines"that the subculture operation can be performed while currently receiving various signals from the first culture unit 12a and determines that the subculture operation cannot be performed while currently receiving various signals from the second culture unit 12b. Then, when the subculture operation can be performed, the CPU 101a performs the subculture operation in step S21, while when the operation cannot be performed, the CPU 101a performs step S22.

In step S22, the CPU 101a displays completion of a monolayer culture operation on a monitor 104. The display is continued until a termination command or correction command is supplied to the CPU 101a by means of a keyboard 103. In response to the termination command, the CPU 101a stops all processes and turns the power off. On the other hand, when the correction command is supplied, the CPU 101a follows the correction command.

In the subculture operation in step S21, the CPU 101a communicates the first liquid discharge pipe 33a with the liquid discharge pipe 81 so as to discharge a waste culture medium 47 in the first culture chamber 46a to the liquid waste tank 16 and also extends the rotating bar 25a. Upon completion of discharge of the waste culture medium 47, the CPU 101a closes the fourth electrically driven valve 82 and puts the stage plate 23 to a horizontal position. The CPU 101a switches the first to third electrically driven valves 54, 70 and 73 so as to communicate a release agent pipe 69a, a subculture pipe 71, a liquid feed pipe 72 and the first liquid feed pipe 32a with each other and also drives the liquid feed pump 74 so as to feed a cell release agent in the release agent tank 62 to the first culture chamber 46a. Since the cell release agent is heated when passing through a heating portion 71a, activity of the cell release agent is increased and the cell release agent does not shock cells in the first culture chamber 46a by its temperature. When a predetermined amount of the cell release agent is fed, the CPU 101a closes the first and third electrically driven valves 54 and 73.

The CPU 101a determines it based on the partial section data whether adhered cells have been detached from the hydrophilic film 43. When few adhered cells remain, the CPU 101a feeds a release agent inhibitor to the first culture chamber 46a. More specifically, the CPU 101a switches the first to third electrically driven valves 54, 70 and 73 so as to communicate a release agent inhibitor pipe 69b, the subculture pipe 71, the liquid feed pipe 72 and the first liquid feed pipe 32a with each other. The CPU 101a also drives the liquid feed pump 74 so as to feed a release agent inhibitor in the release agent inhibitor tank 63 to the first culture chamber 46a and waits for a predetermined amount of time. The CPU 101a switches the first and third electrically driven valves 54 and 73 so as to communicate the second culture medium feed pipe 67b, the liquid feed pipe 72 and the first liquid feed pipe 32a with each other. To prepare a cell suspension, the CPU 101a drives the liquid feed pump 74 so as to feed a hot culture medium 47 from the hot culture medium tank 65 to the first culture chamber 46a.

The CPU 101a switches the fourth electrically driven valve 82 so as to communicate the first and second liquid discharge pipes 33a and 33b and also drives the cell transfer pump 84 so as to transfer a cell suspension in the first culture chamber 46a to the second culture chamber 46b. At the same time, the CPU 101a inclines the stage plate 23 of the first culture unit 12a. The CPU 101a stops the cell transfer pump 84 and, at the same time, closes the fourth electrically driven valve 82.

The CPU 101a not only activates the CCD camera 22 of the second culture unit 12b, the heat retaining plate 26 and the lighting unit 107 but also switches from an input signal from the CCD camera 22 of the first culture unit 12a to an input signal from the CCD camera 22 of the second culture unit 12b. Further, the CPU 101a switches the fifth and sixth electrically driven valves 95 and 97 so as to feed gas into the culture container 31 of the second culture unit 12b. Then, the CPU 101a returns to step S3 and performs a process which is similar to that performed in the first culture unit 12a in the second culture unit 12b.

When the culture medium replacing operations in steps S9 and S20 are to be performed in the second culture unit 12b, the CPU 101a firstly switches the fourth electrically driven valve 82 so as to communicate the second liquid discharge pipe 33b with the liquid discharge pipe 81, drives the liquid discharge pump 83 so as to discharge the waste culture medium 47 in the second culture chamber 46b to the liquid waste tank 16, and inclines the stage plate 23. Then, upon completion of discharge of the waste culture medium 47 in the second culture chamber 46b, the CPU 101a closes the fourth electrically driven valve 82 and puts the stage plate 23 to a horizontal position. Then, the CPU 101a switches the first and third electrically driven valves 54 and 73 so as to communicate the second culture medium feed pipe 67b, the liquid feed pipe 72 and the second liquid feed pipe 32b with each other, then drives the liquid feed pump 74 so as to feed the hot culture medium 47 in the hot culture medium tank 65 to the second culture chamber 46b, and then closes the first and third electrically driven valves 54 and 73.

Therefore, the cell culture device 11 and culture method of the first embodiment have the following advantages.

Image data of cells in the culture container 31 is provided from the CCD camera 22 to the control unit 101. The control unit 101 calculates adhered cell concentration based on the image data, determines timing to perform a culture medium replacing operation and a subculture operation based on the result of the calculation and activates the cell culture device 11 in accordance with the timing. Thereby, since the control unit 101 can accurately monitor statuses of cells, efficient culture operations can be performed.

Since almost all culture operations are performed automatically, efforts, time and costs required for culturing cells are reduced.

Timing to perform the culture medium replacing operation in step S9 is determined based on an increase rate of adhered cell concentration. Therefore, more cells are adhered.

Since time during which a cell release agent and a release agent inhibitor are in contact with cells is reduced, cytotoxic effects of the cell release agent and release agent inhibitor are reduced. In other words, damages caused on cells by the cell release agent and the release agent inhibitor at the time of performing the subculture operation can be reduced.

Since timing to perform the culture medium replacing operation is determined based on a calculated cumulative culture medium consumption, wastage of the culture medium 47 is reduced. Therefore, cells are cultured efficiently.

The culture container 31 is assembled without any germs included therein, and cells, a culture medium and gas are supplied to the culture container 31 without any germs involved. Therefore, a possibility that germs may be mixed into cells during culture of the cells is reduced. As for a method for assembling a germ-free culture container 31, it includes a method in which the culture container 31 is assembled under a germ-free atmosphere and a method in which the culture container 31 is sterilized after assembled.

### (Second Embodiment)

A cell culture device 11 and a method for culturing cells according to a second embodiment of the present invention will be described by giving mainly the points which are different from the first and second embodiments.

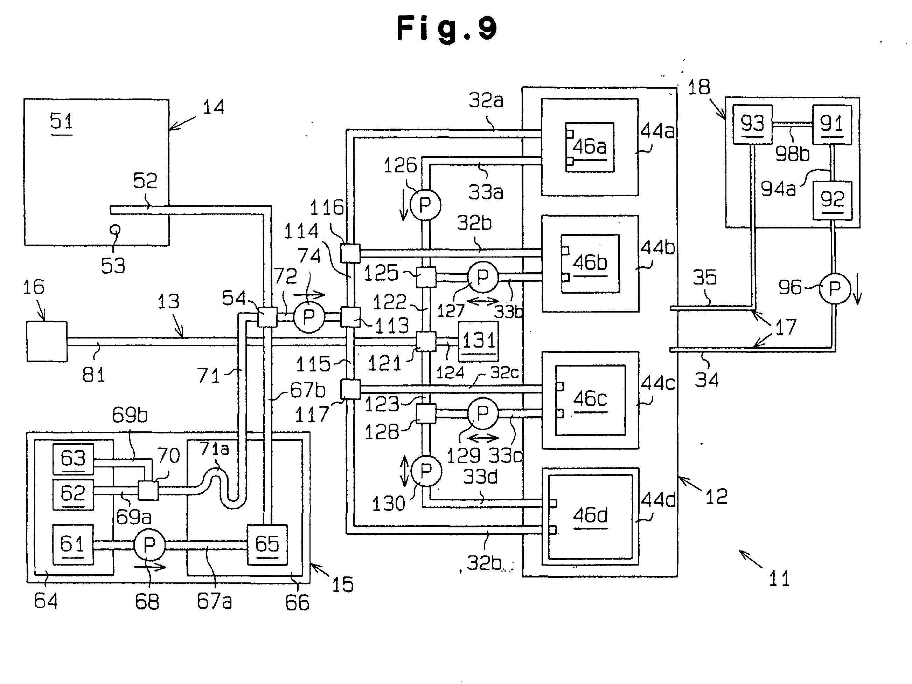

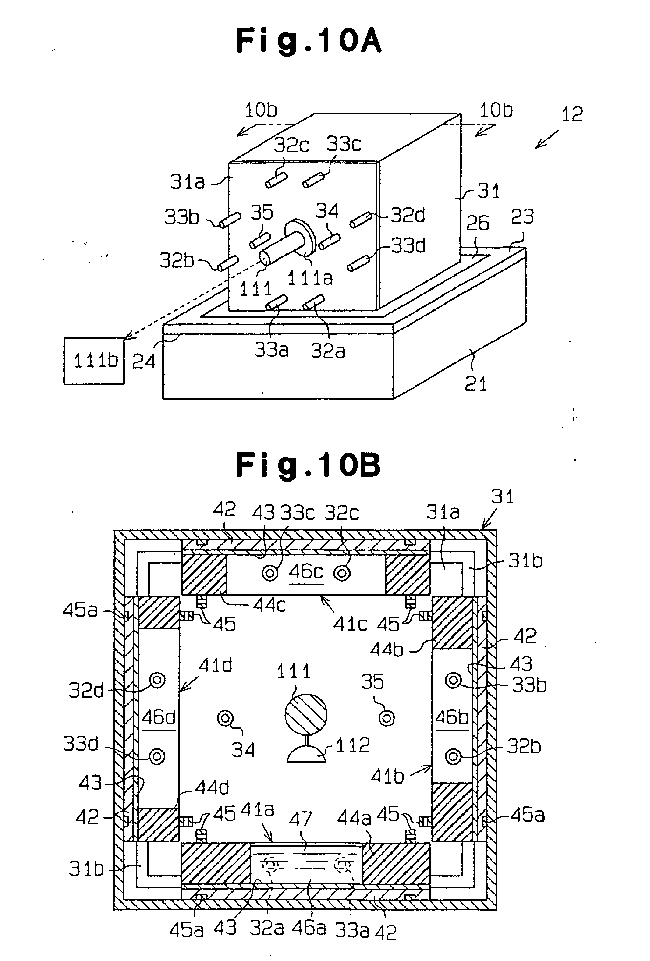

As shown in Fig. 9, the culture device 11 has one culture unit 12. As shown in Fig. 10, the culture unit 12 includes a culture container 31. The culture container 31 is preferably formed from a transparent synthetic resin. The culture container 31 is inclined on a hinge 24 which serves as a fulcrum. The culture container 31 has a tube form having openings on the side closer to the hinge 24 and its opposite side. The openings are sealed by covers 31a. As shown in Fig. 10B, on an internal surface of the cover 31a, a projection 31b whose four sides are continuous is formed.

As shown in Fig. 10A, a rotation shaft 111 extends such that it penetrates the culture container 31. More specifically, the rotation shaft 111 is fixed to a cover 31a by means of a pair of nuts 111a. The position of the culture container 31 is finely adjusted in an axial direction of the rotation shaft 111 by adjusting the positions of the nuts 111a. The rotation shaft 111 is connected to a rotating base end 111b which is located on the hinge 24 side of the culture unit 12. The rotating base end 111b rotates the rotation shaft 111 around its axis and inclines the rotation shaft 111 upward. In other words, the culture container 31 and the rotation shaft 111 are inclined at a predetermined angle and rotated, for instance, counterclockwise 90° at a time, by the rotating base end 111b. The rotating base end 111b includes, for example, a motor, gear and hinge mechanism. Further, as shown in Fig. 10B, a lighting unit 112 for constantly lighting the bottom of the culture container 31 is disposed in the middle of the longitudinal axis of the rotation shaft 111.

As shown in Fig. 10A, along four edges of the cover 31a, a first liquid feed pipe 32a, a first liquid discharge pipe 33a, a second liquid feed pipe 32b, a second liquid discharge pipe 33b, a third liquid feed pipe 32c, a third liquid discharge pipe 33c, a fourth liquid feed pipe 32d and a fourth liquid discharge pipe 33d are connected clockwise. To the covers 31a on both sides of the rotation shaft 111, a gas feed pipe 34 and a gas discharge pipe 35 are connected.

As shown in Fig. 10B, to four internal surfaces of the culture container 31 which surround the rotation shaft 111, a first culture dish 41a, a second culture dish 41b, a third culture dish 41c and a fourth culture dish 41d are bonded. The first and second culture dishes 41a and 41b are the same as those in the first embodiment. The third culture dish 41c includes a third barrier 44c which partitions a third culture chamber 46c having a larger base area than a base area of a second culture chamber 46b. The fourth culture dish 41d includes a fourth barrier 44d which partitions a fourth culture chamber 46d having a larger base area than a base area of a third culture chamber 46c of the third culture dish 41c.

As shown in Fig. 9, a liquid feed pipe 72 connects a first electrically driven valve 54 to a seventh electrically driven valve 113. The seventh electrically driven valve 113 is connected to a first liquid distribution pipe 114 and a second liquid distribution pipe 115. The first liquid distribution pipe 114 is connected to an eighth electrically driven valve 116. The eighth electrically driven valve 116 is connected to the first liquid feed pipe 32a and the second liquid feed pipe 32b. The second liquid distribution pipe 115 is connected to a ninth electrically driven valve 117. The ninth electrically driven valve 117 is connected to the third liquid feed pipe 32c and the fourth liquid feed pipe 32d.

A liquid discharge pipe 81 is connected to a liquid waste tank 16 and a tenth electrically driven valve 121. The tenth electrically driven valve 121 is connected to a first liquid transfer pipe 122, a second liquid transfer pipe 123 and a third liquid transfer pipe 124. The first liquid transfer pipe 122 is connected to an eleventh electrically driven valve 125. The eleventh electrically driven valve 125 is connected to the first liquid discharge pipe 33a and the second liquid discharge pipe 33b. Further, a first cell transfer pump 126 is provided in the first liquid discharge pipe 33a, and a second cell transfer pump 127 which is capable of transferring cells in both directions is provided in the second liquid discharge pipe 33b.

The second liquid transfer pipe 123 is connected to a twelfth electrically driven valve 128. The twelfth electrically driven valve 128 is connected to the third liquid discharge pipe 33c and the fourth liquid discharge pipe 33d. In the middle of the third liquid discharge pipe 33c and the fourth liquid discharge pipe 33d, a third cell transfer pump 129 and a fourth cell transfer pump 130 which are capable of transferring cells in both directions are provided, respectively. Further, the third liquid transfer pipe 124 is connected to a cell storage tank 131 for storing a cell suspension temporarily at the time of subculture.

A gas exchange unit 18 includes a humidifier 92 and a gas analyzer 93. The humidifier 92 is connected to the gas feed pipe 34 having a gas circulating pump 96, and the gas analyzer 93 is connected to the gas discharge pipe 35.

Other constituents are the same as those in the first embodiment.

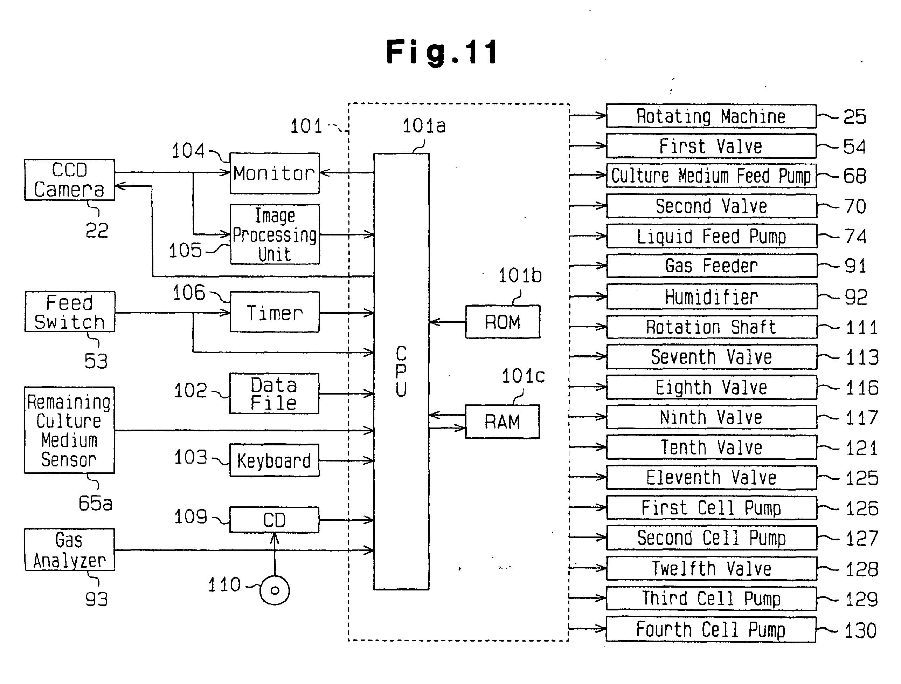

As shown in Fig. 11, in the second embodiment, a CPU 101a controls the rotation shaft 111. More specifically, the CPU 101a inclines the rotation shaft 111 at a given angle at the rotating base end 111b and rotates the culture container 31 90° at the rotation shaft 111. Thereafter, the CPU 101a puts the rotation shaft 111 back to a horizontal position and puts the culture container 31 on the stage plate 23. The CPU 101a switches the seventh electrically driven valve 113 so as to communicate the liquid feed pipe 72 with the first liquid distribution pipe 114 or the second liquid distribution pipe 115. The CPU 101a switches the eighth electrically driven valve 116 so as to communicate the first liquid distribution pipe 114 with the first liquid feed pipe 32a or the second liquid feed pipe 32b. The CPU 101a switches the ninth electrically driven valve 117 so as to communicate the second liquid distribution pipe 115 with the third liquid feed pipe 32c or the fourth liquid feed pipe 32d.

The CPU 101a switches the tenth electrically driven valve 121 so as to communicate the first liquid transfer pipe 122 with the liquid discharge pipe 81 or communicate the second liquid transfer pipe 123 with the liquid discharge pipe 81 or communicate the first liquid transfer pipe 122 with the third liquid transfer pipe 124 or communicate the third liquid transfer pipe 124 with the second liquid transfer pipe 123. The CPU 101a switches the eleventh electrically driven valve 125 so as to communicate the first liquid discharge pipe 33a with the first liquid transfer pipe 122 or communicate the second liquid discharge pipe 33b with the first liquid transfer pipe 122.

The CPU 101a sends a drive signal to the first cell transfer pump 126 so as to pump a liquid (waste culture medium 47 or cell suspension) in the first culture chamber 46a and transfer the liquid to the liquid waste tank 16 or cell storage tank 131. The CPU 101a drives the second cell transfer pump 127 so as to pump a liquid (waste culture medium 47 or cell suspension) in the second culture chamber 46b and transfer the liquid to the liquid waste tank 16 or cell storage tank 131 and to transfer a cell suspension in the cell storage tank 131 to the second culture chamber 46b.

The CPU 101a switches the twelfth electrically driven valve 128 so as to communicate the third liquid discharge pipe 33c with the second liquid transfer pipe 123 or communicate the fourth liquid discharge pipe 33d with the second liquid transfer pipe 123. The CPU 101a drives the third cell transfer pump 129 so as to pump a liquid (waste culture medium 47 or cell suspension) in the third culture chamber 46c and transfer the liquid to the liquid waste tank 16 or cell storage tank 131 and to transfer the cell suspension in the cell storage tank 131 to the third culture chamber 46b. The CPU 101a drives the fourth cell transfer pump 130 so as to pump a liquid (waste culture medium 47 or cell suspension) in the fourth culture chamber 46d and transfer the liquid to the liquid waste tank 16 or cell storage tank 131 and to transfer the cell suspension in the cell storage tank 131 to the fourth culture chamber 46d.

To a control unit 101 of the second embodiment, a heat retaining plate 26, a lighting unit 107, a third electrically driven valve 73, a fourth electrically driven valve 82, a liquid discharge pump 83, a cell transfer pump 84, a firth electrically driven valve 95 and a sixth electrically driven valve 97 are not connected.

Use of the cell culture device 11 will be described hereinafter.

To culture anchorage-dependent cells, the first to fourth culture dishes 41a, 41b, 41c and 41d are assembled in the same manner as in Example 1 without any germs involved. Engagement lugs 45d of the first to fourth culture dishes 41a, 41b, 41c and 41d are caused to engage corresponding projections 31b of the culture container 31. The covers 31a are attached to the culture container 31. The rotation shaft 111 is caused to penetrate the culture container 31. The surface on which the first culture dish 41a is fixed is placed on the heat retaining plate 26. The culture container 31 is fixed to the rotation shaft 111 by nuts 111a. Then, sufficient amounts of culture medium 47, cell release agent, release agent inhibitor, carbon dioxide gas, oxygen gas, and water are prepared. Then, an operator commands the CPU 101a to start culturing by means of the keyboard 103.

In response to the start command, the CPU 101a performs the same processes as those in the first embodiment. More specifically, in step S1, the CPU 101a checks an operation environment of the cell culture device 11. The CPU 101a activates the CCD camera 22, heat retaining plate 26, lighting unit 112, simple refrigerator 64, incubator 66, gas analyzer 93, gas circulating pump 96 and remaining culture medium sensor 65a. Thereby, the lighting unit 112 lights the first culture dish 41a, the CCD camera 22 photographs the first culture chamber 46a, and the picture is displayed on the monitor 104. As in the case of the first embodiment, data about the operation environment of the cell culture device 11 is displayed on the monitor 104. A predetermined amount of the culture medium 47 is fed to the hot culture medium tank 65. Gas adjusted based on data on gas analyzed by the gas analyzer 93 is fed to the culture container 31 by the gas circulating pump 96. The CPU 101a displays a message notifying that everything is ready for starting culturing on the monitor 104.

In step S2, cells are inoculated. More specifically, the operator immerses the tip of the cell feed pipe 52 in a cell suspension prepared in the clean bench 51 and presses down the feed switch 53. In response to this, the CPU 101a switches the first, seventh and eighth electrically driven valves 54, 113 and 116 so as to communicate the cell feed pipe 52, the liquid feed pipe 72, the first liquid distribution pipe 114 and the first liquid feed pipe 32a with each other. The CPU 101a drives the liquid feed pump 74 so as to feed the cell suspension to the first culture chamber 46a. Thereafter, the control unit 101 carries out routines in steps S3 to S8.

In a culture medium replacing operation in step S9, the CPU 101a switches the tenth and eleventh electrically driven valves 121 and 125 so as to communicate the first liquid discharge pipe 33a, the first liquid transfer pipe 122 and the liquid discharge pipe 81 with each other. Thereafter, the CPU 101a drives the first cell transfer pump 126 so as to discharge a waste culture medium 47 in the first culture chamber 46a to the liquid waste tank 16 and inclines the stage plate 23. Upon completion of discharge of the waste culture medium 47, the CPU 101a closes the eleventh electrically driven valves 125 and puts the stage plate 23 back to a horizontal position. The CPU 101a switches the first, seventh and eighth electrically driven valves 54, 113 and 116 so as to communicate the second culture medium feed pipe 67b, the liquid feed pipe 72, the first liquid distribution pipe 114 and the first liquid feed pipe 32a with each other. Thereafter, the CPU 101a drives the liquid feed pump 74 so as to feed a hot culture medium 47 from the hot culture medium tank 65 to the first culture chamber 46a. The CPU 101a closes the first, seventh and eighth electrically driven valves 54, 113 and 116 at the same time it stops the liquid feed pump 74.

Then, the CPU 101a carries out routines in steps S10 to S15. In step S15, the CPU 101a compares adhered cell concentration stored in the RAM 101c with a preset value (threshold value) stored in the data file 102. When the adhered cell concentration is lower than the threshold value, step S16 and steps S18 to S20 are carried out. A culture medium replacing operation in step S20 is the same as that in step S9 of the second embodiment. When the adhered cell concentration is higher than or equal to the threshold value, step S17 is carried out.

In step S17, the CPU 101a checks in which of the first to fourth culture dishes 41a, 41b, 41c and 41d anchorage-dependent cells are currently cultured and also determines whether a subculture operation is possible. For example, when the anchorage-dependent cells are currently being cultured in the first to third culture dishes 41a, 41b and 41c, the subculture operation is possible. Meanwhile, when the anchorage-dependent cells are currently being cultured in the fourth culture dish 41d, the subculture operation is impossible.

When the subculture operation is possible, the CPU proceeds to step S21, while when the subculture operation is impossible, the CPU proceeds to step S22.

In step S21, the subculture operation is performed. As in the case of the culture medium replacing operation, the CPU 101a firstly communicates the first liquid discharge pipe 33a, the first liquid transfer pipe 122 and the liquid discharge pipe 81 with each other so as to discharge a waste culture medium 47 from the first culture chamber 46a to the liquid waste tank 16 and also inclines the stage plate 23. Upon completion of discharge of the waste culture medium 47, the CPU 101a closes the eleventh electrically driven valve 125 and puts the stage plate 23 back to a horizontal position. The CPU 101a switches the first, second, seventh and eighth electrically driven valves 54, 70, 113 and 116 so as to communicate the release agent pipe 69b, the subculture pipe 71, the liquid feed pipe 72, the first liquid distribution pipe 114 and the first liquid feed pipe 32a with each other. The CPU 101a drives the liquid feed pump 74 so as to feed a cell release agent from the release agent tank 62 to the first culture chamber 46a. Thereafter, the CPU 101a closes the first, second, seventh, and eighth electrically driven valves 54, 70, 113, and 116.

The CPU 101a examines partial section data entered via the CCD camera 22. When an image of cells on the hydrophilic film 43 becomes hardly recognizable, the CPU 101a feeds a release agent inhibitor to the first culture chamber 46a. More specifically, the CPU 101a firstly switches the first, second, seventh and eighth electrically driven valves 54, 70, 113 and 116 so as to communicate the release agent inhibitor pipe 69b, the subculture pipe 71, the liquid feed pipe 72, the first liquid distribution pipe 114 and the first liquid feed pipe 32a with each other. The CPU 101a drives the liquid feed pump 74 so as to feed a release agent inhibitor from the release agent inhibitor tank 63 to the first culture chamber 46a. After passage of predetermined time, the CPU 101a switches the first, seventh and eighth electrically driven valves 54, 113 and 116 so as to communicate the second culture medium feed pipe 67b, the liquid feed pipe 72, the first liquid distribution pipe 114 and the first liquid feed pipe 32a with each other. The CPU 101a drives the liquid feed pump 74 so as to feed a hot culture medium 47 from the hot culture medium tank 65 to the first culture chamber 46a. Thereby, a cell suspension is prepared.

The CPU 101a switches the tenth and eleventh electrically driven valves 121 and 125 so as to communicate the first liquid discharge pipe 33a, the first liquid transfer pipe 122 and the third liquid transfer pipe 124 with each other. The CPU 101a drives the first cell transfer pump 126 so as to feed a cell suspension in the first culture chamber 46a to the cell storage tank 131 and also inclines the stage plate 23. The CPU 101a closes the eleventh electrically driven valve 125 at the same time it stops the first cell transfer pump 126.

Then, the CPU 101a inclines the rotation shaft 111 at a given angle and rotates the rotation shaft 111 90° around its axis. Thereby, the culture container 31 is rotated 90°. Thereafter, the CPU 101a puts the rotation shaft 111 back to a horizontal position and mounts the culture container 31 on the stage plate 23. By rotation of the culture container 31, the second culture dish 41b comes to a low position. Therefore, cells are cultured in the second culture chamber 46b.

The CPU 101a switches the tenth and eleventh electrically driven valves 121 and 125 so as to communicate the second liquid discharge pipe 33b, the first liquid transfer pipe 122 and the third liquid transfer pipe 124 with each other. The CPU 101a drives the second cell transfer pump 127 so as to feed a cell suspension in the cell storage tank 131 to the second culture chamber 46b. The CPU 101a closes the eleventh electrically driven valve 125 at the same time it stops the second cell transfer pump 127. Then, the CPU 101a carries out subsequent steps including step S3 on anchorage-dependent cells in the second culture chamber 46b.

Anchorage-dependent cells are subcultured in the second culture chamber 46b, the third culture chamber 46c and the fourth culture chamber 46d in turn by the cell culture device 11. When the CPU 101a determines it in a culture step in the fourth culture chamber 46d that no further subculture operation is possible (step S17), step S22 is carried out.

When a cell suspension is transferred between subculture operations, the cell suspension is stored in the cell storage tank 131 temporarily. Then, after the culture container 31 is rotated, the cell suspension is transferred from the cell storage tank 131 to a new culture dish. To feed a liquid into a culture dish in the culture medium replacing operation and the subculture operation, the CPU 101a selectively drives a pump provided between a tank containing the target liquid and the culture dish. The same applies to when the liquid is discharged from the culture dish.

Thus, the same effects as those attained by the first embodiment can be attained by the cell culture device 11 and culture method of the second embodiment.

The cell culture device 11 is relatively small and can perform more subculture operations. Particularly, when a large amount of cells are cultured by a number of subculture operations, a tubular culture container 31 which has a number of side faces is used. Therefore, it is not necessary to increase the number of the culture container 31. In this case, by changing the number of culture dishes and placement of the liquid pipe 13, complex and expensive members such as the stage 21 and the CCD camera 22 remain intact.

The embodiments may be modified in the following manner.

As an indicator for indicating density of cells adhered to the hydrophilic film 43, the number of the adhered cells, an area occupied by the adhered cells, or a proportion of the area occupied by the adhered cells to an area of the hydrophilic film 43 to which cells can possibly adhere can be used in place of concentration of the adhered cells.

In the culture container 31, a sensor for measuring an amount of given component (preferably the most consumable components or components which are easy to measure, such as glutamine, glutamate, glucose and lactate) remaining in the culture medium 47 may be provided. In this case, in place of a cumulative culture medium consumption calculated in step S16, timing to perform a culture medium replacing operation is determined based on an amount of component measured by the sensor. Therefore, the timing to perform the culture medium replacing operation is determined accurately.

A vibrator to vibrate the culture container 31 may be provided on the underside of the stage plate 23. In this case, cells are detached more easily by vibrating the culture container 31 during a subculture operation, for example, a cell detaching step.

A cleaning agent tank for storing a calcium-ion-free isotonic solution for cleaning such as a calcium-ion-free phosphate buffer or a calcium-ion-free serum-free culture medium may be connected to the second electrically driven valve 70. Further, an operation in which a cleaning isotonic solution is fed into and pumped out of a culture chamber having some cells adhered therein may be performed immediately after a waste culture medium 47 is discharged at the time of subculturing so as to remove calcium ions in the culture chamber. In this case, the cells are detached smoothly.

A release agent inhibitor may not be used at the time of subculturing. In this case as well, activity of a cell release agent can be suppressed by calcium ions contained in a culture medium 47 so as to adhere cells onto the hydrophilic film 43. Further, a cytotoxic effect by the release agent inhibitor is reduced.

In the second embodiment, the criterion in step S17 may be the number of processed culture dishes entered in advance by means of the keyboard 103. In this case, desired culture conditions can be obtained easily.

The culture container 31 in the second embodiment may be changed to a polygonal tube such as a triangular tube, hexagonal tube or an octagonal tube. In this case, the number of culture dishes is changed to 3, 6 or 8. According to the shape of the culture container 31, a desired number of subculture operations are performed.

The base plate 42 in the second embodiment may be omitted, and the hydrophilic film 43 and the first to fourth barriers 44a, 44b, 44c and 44d may be fixed to the four internal surfaces of the culture container 31. In this case, the culture container 31 is simplified.

In the second embodiment, the stage plate 23 and the rotating machine 25 may be omitted. Further, it is preferred that the heat retaining plate 26 be provided on the undersides of the culture dishes 41a, 41b, 41c and 41d or gas having a predetermined temperature (for example 37°C) be supplied from the gas exchange unit 18. In this case, the cell culture device 11 is simplified. Further, liquids in the culture chambers are removed efficiently.

In the second embodiment, the rotation shaft 111 may be slid upward in a horizontal position without being inclined. Alternatively, the stage plate 23 may be slid. In this case, the culture container 31 can be rotated easily.

The embodiments may be constituted such that operation environment data of the cell culture device 11 can be viewed on the Internet. Further, the control unit 101 may be controlled via the Internet. In this case, statuses of cells being cultured can be checked easily at a remote site.

The cell culture device 11 in the first embodiment may be constituted only by the first culture unit 12a or the second culture unit 12b and the control unit 101. Alternatively, the cell culture device 11 in the second embodiment may be constituted only by the culture unit 12 and the control unit 101. In this case, the cell culture device 11 is simplified. Further, when a screen for directing an operator to perform a culture operation is displayed on the monitor 104 according to cell status captured by the CCD camera 22, the operator can realize timing to perform the culture operation easily.

The cell culture device 11 in the first embodiment may be constituted such that the second culture unit 12b, the release agent tank 62, the release agent inhibitor tank 63, the pipes connected to the unit and tanks, the electrically driven valves and the pumps are omitted and only the culture medium replacing operation is performed automatically. Further, the cell feed unit 14 may also be omitted. In this case, the culture medium replacing operation can be performed automatically while the constitution of the cell culture device 11 is simplified.

In step S6, an increase curve or increase straight line of an increase rate of adhered cell concentration may be estimated by the CPU 101a based on the calculated adhered cell concentration so as to display timing to perform the post-adhesion culture medium replacing operation in step S9 on the monitor 104 based on the result of the estimation. In this case, since an amount of time required to culture cells can be estimated easily, a schedule for culturing can be adjusted easily.

An increase curve or increase straight line of a cumulative culture medium consumption may be estimated by the CPU 101a based on calculated culture medium consumption so as to display timing to perform the culture medium replacing operation in step S20 on the monitor 104 based on the result of the estimation. In this case, since an amount of time required to culture cells can be estimated easily, a schedule for culturing can be adjusted easily.

In step S14, an increase curve or increase straight line of adhered cell concentration may be estimated by the CPU 101a based on the calculated adhered cell concentration so as to display timing to perform the subculture operation in step S21 on the monitor 104 based on the result of the estimation. In this case, since an amount of time required to culture cells can be estimated easily, a schedule for culturing can be adjusted easily.

A scanner may be incorporated into the CCD camera 22 so as to photograph cells with a focal depth of the lens 22a being changed continuously. Further, a total number or concentration of cells in a cell suspension inoculated in the culture container 31 may be determined from photographed image data. In this case, when inoculated cell concentrations are calculated at the times of a cell inoculation operation and a subculture operation, an adhesion rate curve indicating a relationship between culture time and an adhesion rate of anchorage-dependent cells in a cell adhesion period (stage from inoculation of the cells in the culture chamber and adhesion of the cells to the bottom of the culture chamber) can be determined by use of the concentrations.

Further, the CPU 101a may estimate behaviors of cells of the same type during the cell adhesion period and outputs the result of the estimation to output means at the times of the cell inoculation operation and the subculture operation based on an average curve of the adhesion rate curve which is prepared and stored in the data file 102 and the calculated inoculated cell concentration. Further, it is preferred that an adhesion rate curve be prepared every time cells of the same type are cultured so as to correct the average curve stored in the data file 102. In this case, since an amount of time required to culture cells can be estimated easily, a schedule for culturing can be adjusted easily.

The embodiments may be constituted such that the CPU 101a calculates a lag time indicating a length of a lag phase (stage from end of the cell adhesion period to start of divisions of adhered cells) based on calculated adhered cell concentration in the lag phase and then estimates behaviors of cells of the same type in the lag phase and outputs the result of the estimation to output means based on an inoculated cell concentration and the lag time. Further, it is preferred that a difference between an estimation result and a measured value be corrected every time cells of the same type are cultured so as to be able to use the results in the next estimation. In this case, a schedule for culturing can be adjusted easily.

The embodiments may be constituted such that the CPU 101a calculates an average doubling time or apparent doubling time indicating intervals of cell divisions based on calculated adhered cell concentration in a logarithmic growth phase (stage from end of the lag phase until cells become nearly confluent state) and then estimates an amount of time required for the cells to become confluent state and outputs the result of the estimation to output means based on the result of the calculation. Further, it is preferred that a difference between an estimation result and a measured value be corrected every time cells of the same type are cultured so as to be able to use the results in the next estimation. In this case, a schedule for culturing can be adjusted easily.

An adhesion rate curve, a lag time and an average doubling time or apparent doubling time may be estimated based on inoculated cell concentration so as to estimate adhered cell concentration in each culture time (particularly after the logarithmic growth phase) and output the result of the estimation to output means. In this case, since an amount of time required to culture cells can be estimated easily, a schedule for culturing can be adjusted easily.

Anchorage-dependent cells may be subjected to tissue culture (multilayered culture or three-dimensional culture) by use of the cell culture device 11 of the embodiment. That is, a correction command may be given to the CPU 101a by means of the keyboard 103 so that cells which have become confluent state in the second culture chamber 46b of the first embodiment or the fourth culture chamber 46d of the second embodiment are further cultured in the culture chamber by replacing a culture medium in the culture chamber with a culture medium 47 for tissue culture as required. In this case, cultured tissue 140 of desired size can be obtained easily by use of the cell culture device 11.

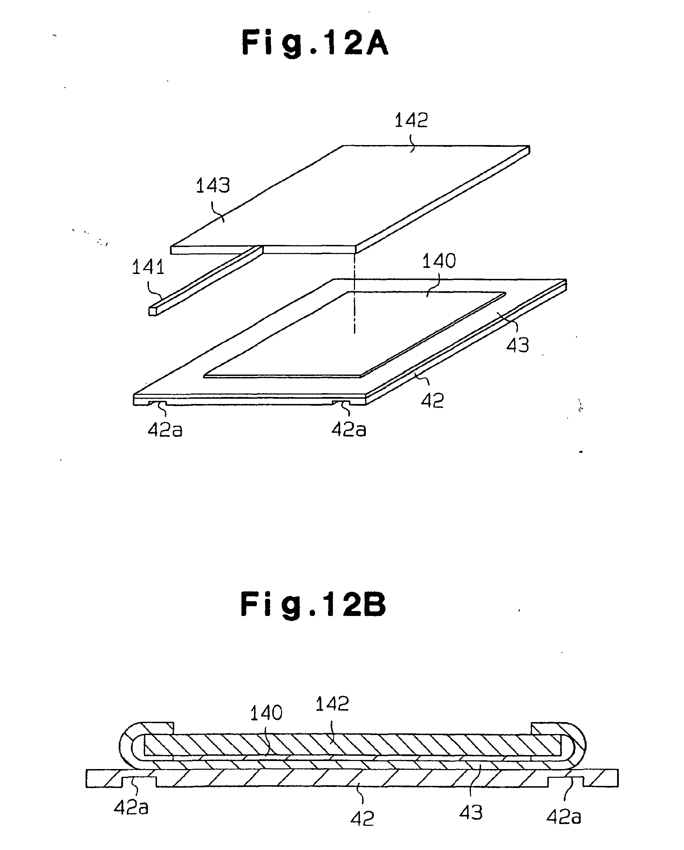

To extract the cultured tissue, firstly, as shown in Fig. 12A, the second barrier 44b or the fourth barrier 44d is removed from the second culture dish 41b of the first embodiment or the fourth culture dish 41d of the second embodiment, and the base plate 42, the hydrophilic film 43 and the cultured tissue 140 are taken out. Thereafter, by use of a tissue extracting jig 143 which comprises a stick handle 141 and a contact plate 142, the cultured tissue 140 is separated from the base plate 42 and the hydrophilic film 43. That is, an operator grasps the handle 141 so as to bring the contact plate 142 into intimate contact with the top surface of the cultured tissue 140. As shown in Fig. 12B, edges of the hydrophilic film 43 are folded onto the top surface of the contact plate 142. The operator grasps the handle 141 so as to lift the contact plate 142, the cultured tissue 140 and the hydrophilic film 43, thereby separating the base plate 42 from the hydrophilic film 43. The hydrophilic film 43 is removed from the cultured tissue 140 carefully, thereby obtaining the cultured tissue 140 closely adhered to the contact plate 142.

## Claims

1. A method for monolayer-culturing anchorage-dependent cells in a culture container, comprising the steps of:
photographing anchorage-dependent cells in a culture container and producing image data;
calculating at least one status value of the number of adhered cells in the culture container, the concentration of the adhered cells, an area occupied by the adhered cells and an occupancy rate of the adhered cells based on the image data;
determining timing to perform at least one operation selected from a culture medium replacing operation and a subculture operation based on the at least one status value; and
performing the selected operation at the determined timing.

2. The method according to claim 1, wherein the step of performing the selected subculture operation comprises:
a step of adding a cell release agent to the culture container so as to detach cells adhered to the bottom of the culture container;
a step of preparing a cell suspension while terminating the reaction to detach the cells;
a step of distributing the cell suspension over a plurality of culture containers; and
a post-adhesion culture medium replacing step of replacing a culture medium after cells in the distributed cell suspension are adhered to the bottom of a corresponding culture container, the post-adhesion culture medium replacing step being carried out when an increase rate of any one of the status values becomes smaller than a predetermined value.

3. The method according to claim 1 or 2, further comprising the steps of:
calculating a culture medium consumption in a predetermined culture period and a cumulative culture medium consumption based on any one of the status values; and
performing the culture medium replacing operation when the cumulative culture medium consumption reaches a predetermined value.

4. A cell culture device (11) for monolayer-culturing anchorage-dependent cells in a culture container while performing at least one operation selected from a culture medium replacing operation and a subculture operation, the cell culture device comprising:
a culture container (31);
a photographic unit (22) for photographing an image of anchorage-dependent cells in the culture container so as to produce image data of the cells; and
a control unit (101) which is connected to the photographic unit so as to process the image data; and
the control unit calculating at least one of the number of cells adhered in the culture container, the concentration of the cells adhered in the culture container, an area in the culture container which is occupied by the adhered cells, and a proportion of the adhered cells in the culture container; and determining timing to perform the operation based on the result of the calculation.

5. The cell culture device according to claim 4, wherein the culture container has a plurality of culture dishes (41a, 41b, 41c, 41d) including a first culture dish (41a) and a second culture dish (41b, 41c, 41d), wherein the first culture dish has a first area and the second culture dish has a second area which is larger than the first area, the cell culture device further comprises:
a culture medium feed device (54, 61, 65, 68, 73, 74) for feeding a culture medium (47) to each of the culture dishes;
a release agent feed device (62, 70, 54, 73, 74) for feeding a cell release agent to each of the culture dishes;
a liquid discharge device (82, 83) for discharging a liquid from each of the dishes; and
a cell transfer device (82, 84) for transferring cells from the first culture dish to the second culture dish.

6. The cell culture device according to claim 5, wherein the culture container is a polygonal tubular container which has a plurality of side faces and a shaft (111) extending almost horizontally, the plurality of culture dishes are located on the inner surfaces of the side faces of the culture container, and the cell culture device further comprises a position changing device (111a) for selectively moving at least one of the culture dishes to a low position.

7. The cell culture device according to any of claims 4 to 6, which further comprises an inclination device (25, 111a) for inclining the culture container at a predetermined angle.

8. The cell culture device according to any of claims 4 to 7, which further comprises a cell feed device (14) for feeding cells placed in a clean bench (51) to the culture container.

9. A system for monolayer-culturing anchorage-dependent cells, comprising:
a first culture dish (41a) which partitions a first culture chamber (46a) in which the cells are cultured;
a second culture dish (41b) which partitions a second culture chamber (46b) connected to the first culture chamber and having a larger base area than that of the first culture chamber;
a photographic unit (22) for photographing cells in the first and second culture chambers so as to produce image data;
a control unit (101, 105) which is connected to the photographic unit so as to process the image data;
a transfer mechanism (33a, 33b, 82, 84, 25, 33c, 33d, 111a, 121, 126, 127, 129, 130, 131) which is connected to the control unit so as to transfer the cells in the first culture chamber to the second culture chamber in accordance with a command from the control unit;
a feed pump (68, 74) which is connected to the control unit so as to selectively feed a culture medium and the cells to the culture chambers in accordance with a command from the control unit; and
a discharge pump (83, 126, 127, 129, 130) which is-connected to the control unit so as to discharge a waste culture medium from the culture chambers in accordance with a command from the control unit; wherein the control unit calculates at least one of the number of cells adhered in the culture container, the concentration of the cells adhered in the culture container, an area in the culture container which is occupied by the adhered cells, and a proportion of the adhered cells in the culture container, and controls the transfer mechanisms, the feed pumps and the discharge pumps based on the result of the calculation.

10. The system according to claim 9, wherein the transfer mechanism comprises:
a transfer pump (84, 126, 127, 129, 130) disposed between the first culture dish and the second culture dish; and
an inclination device (25, 111a) for inclining the first culture dish.

11. A recording medium (109) containing a computer readable program for monolayer-culturing anchorage-dependent cells in a culture container while performing at least one operation selected from a culture medium replacing operation and a subculture operation, wherein the program executes a method comprising the steps of:
inputting image data of anchorage-dependent cells in a culture container by means of a photographic unit;
calculating the number of adhered cells, the concentration of the adhered cells, an area occupied by the adhered cells, or a proportion of the adhered cells based on the image data;
determining timing to perform at least one operation selected from a culture medium replacing operation and a subculture operation based on the result of the calculation; and
performing the operation.

12. A culture container (31) for monolayer-culturing anchorage-dependent cells while performing a subculture operation, wherein the culture container is a polygonal tubular container having a plurality of side faces and a shaft (111) extending nearly horizontally and accommodates a plurality of culture dishes (41a, 41b, 41c, 41d) located on the inner surfaces of the side faces, the culture dishes including a first culture dish (41a) having a first area and second culture dish (41b, 41c, 41d) having a second area which is larger than the first area, and wherein the culture container has a position changing device (111a) for selectively moving one of the culture dishes to a low position.
